(19) Europäisches Patentamt
European Patent Office
Office européen des brevets

(11) Publication number: 0 034 684
B1

(12) EUROPEAN PATENT SPECIFICATION

(45) Date of publication of patent specification: 03.08.83

(21) Application number: 80304603.6

(22) Date of filing: 18.12.80

(51) Int. Cl.[3]: C 07 C 79/18, C 07 C 76/02, A 01 N 37/12, C 02 F 1/50, D 21 H 3/08

# (54) 1,3-Diformyloxy-2-bromo-2-nitropropane, its preparation and use in anti-microbial applications

(30) Priority: 20.02.80 GB 8005801

(43) Date of publication of application: 02.09.81 Bulletin 81/35

(45) Publication of the grant of the patent: 03.08.83 Bulletin 83/31

(84) Designated Contracting States: BE CH DE FR GB IT LI NL SE

(56) References cited:
DE - A - 2 647 392
GB - 874 064
US - A - 2 873 249
US - A - 3 151 020

Beilsteins Handbuch der Organischen Chemie, Edition 4, Vol. 2 pages 165, 166, Verlag von Julius Springer, Berlin, 1920

(73) Proprietor: IMPERIAL CHEMICAL INDUSTRIES PLC
Imperial Chemical House Millbank
London SW1P 3JF (GB)

(72) Inventor: Buckley, Alan John
14 Brookway
Grasscroft Oldham OL4 4EU (GB)

(74) Representative: Pugsley, Roger Graham et al,
IMPERIAL CHEMICAL INDUSTRIES PLC Legal Department: Patents Thames House North Millbank
London SW1P 4QG (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 034 684 B1

# 1,3-Diformyloxy-2-bromo-2-nitropropane, its preparation and use in anti-microbial applications

This invention relates to a new compound having biocidal activity and to its use in the protection of media against infection by micro-organisms.

It is known that 2-bromo-2-nitro-1,3-propanediol (BNPD) and certain di-acyl esters thereof have antimicrobical activity. It has now been found that the diformyloxy ester of BNPD which has not previously been disclosed is also a good anti-microbial and furthermore that it has a fast rate of action especially against the micro-organisms prevalent in industrial aqueous media.

According to the present invention there is provided the compound 1,3-diformyloxy-2-bromo-2-nitro-propane (DFBNP).

This compound may be prepared by the direct esterification of 2-bromo-2-nitro-1,3-propandiol (BNPD) with formic acid in the presence of a strong acid catalyst, such as p-toluene sulphonic acid, but the yield of the diformyl ester from this process is very low and the desired product is contaminated with unreacted material and the mono-ester and thus requires extensive purification. A preferred method is to prepare the mixed anhydride of acetic and formic acids which, though itself unstable, is more stable than either formic anhydride or formyl chloride and to react this immediately with the diol in the presence of a basic catalyst, such as pyridine. Yields in excess of 80% of theory are possible with this process which is a second feature of the present invention.

DFBNP has useful antimicrobial activity and according to a further feature of the invention there is provided a method for the protection of a medium against attack by micro-organisms which comprises adding DFBNP to the medium.

The term micro-organism includes bacteria, fungi and algae and the compound is particularly useful for the preservation of aqueous media from attack by such micro-organisms. As examples of aqueous media there may be mentioned cooling water systems and like enclosed aqueous systems, paper mill liquors ("white water"), and swimming baths and pools. It is however also of value in other applications in which anti-microbials are commonly employed such as for example, preservation of hides, aqueous emulsions, and water-based paints and adhesives.

The quantity of the compound used depends on the nature of the medium and the micro-organism against which attack is being protected but is generally within the range 1 to 5000 parts per million (ppm) and more preferably in the range 10 to 1000 ppm. It is preferred to add the compound in relatively large shock doses at infrequent intervals rather than to maintain a lower constant level in the medium so as to reduce the risk of the development of strains of the micro-organisms which are resistant to the compound. Such shock doses will preferably lie within the above ranges.

The compound may be conveniently added to aqueous media as a formulation in a water-miscible organic liquid (preferably water-soluble to the extent of at least 0.5% by weight). As the compound is susceptible to hydrolysis it is further preferred that the liquid is non-hydroxylic.

The invention is further illustrated by the following Examples in which all parts and percentages are by weight unless otherwise indicated.

## Example 1

Preparation of DFBNP

Formic acid (46.4 parts) is added to 102.8 parts of acetic anhydride with constant stirring causing an exotherm which raises the temperature to 40°C. The reaction mass is held at 40—45°C for a further hour after completion of the exotherm. The temperature is then reduced to 25°C and 0.78 part by pyridine is added followed by 100 parts of BNPD. An endotherm which causes the temperature to fall by 7°C is followed by an exotherm which accelerates above 30°C. A water bath is used to ensure that the temperature does not rise above 40°C. The temperature is allowed to return to ambient over a period of 75 minutes.

The reaction mixture is poured into 200 parts of water, shaken and the two phases allowed to separate. The lower organic layer is drained off and DFBNP in the upper aqueous layer is extracted with chloroform (2 × 149.2 parts), the chloroform extracts are combined with the organic layer. The resulting chloroform solution is dried over anhydrous magnesium sulphate, filtered and evaporated to dryness on a rotary evaporator to yield a clear colourless liquid (107 parts; yield 83.6%). I.R. analysis shows the required C=O stretch at 1719 $cm^{-1}$. N.M.R. analysis shows a single at 2.0 $\tau$ with an integration of 6 attributed to

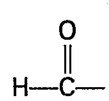

and a singlet at 5.1 $\tau$ with an integration of 12 attributed to

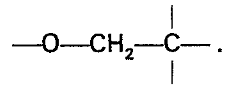

Example 2

The activity of DFBNP against bacteria

A 100 ml volume of a solution of DFBNP containing 50 parts per million (ppm) of biocide in distilled water is prepared and inoculated with 1 ml of an 18 hour broth culture of *Pseudomonas aeruginosa.* A control which is free from biocide is also inoculated in the same fashion. The test solution and control are inspected 1, 2 and 4 hours after inoculation and at each inspection a count of surviving bacterial cells is made. The results are as follows:

| Biocide | Surviving Cells/ml | | |
|---|---|---|---|
| | 1 hour | 2 hours | 4 hours |
| DFBNP | <10 | <10 | <10 |
| Control (no biocide) | $1.8 \times 10^6$ | $3.5 \times 10^7$ | $5.8 \times 10^6$ |

Example 3

Comparison of the activity of DFBNP and Benzisothiazolone (BIT) against bacteria in a paper mill liquor

100 ml volumes of two solutions of each of DFBNP and BIT containing 200 and 500 ppm of biocide in a sterile synthetic white water are prepared and each is inoculated with 1 ml of a 18 hour broth culture of *Enterobacter cloacae.* A control sample of the white water which is free from biocide is inoculated in identical fashion. The test solutions and control are inspected 1, 2 and 4 hours after inoculation and at each inspection a count of surviving bacterial cells is made. The results are as follows:

| | | Surviving Cells/ml | | |
|---|---|---|---|---|
| Biocide | Concentration | 1 hour | 2 hours | 4 hours |
| DFBNP | 200 ppm | <10 | <10 | <10 |
| | 500 ppm | <10 | <10 | <10 |
| BIT | 200 ppm | $9.6 \times 10^5$ | $3.0 \times 10^5$ | <10 |
| | 500 ppm | $2.2 \times 10^3$ | $3.2 \times 10^5$ | <10 |
| Control (no biocide) | | $1.8 \times 10^7$ | $>3.0 \times 10^7$ | $1.2 \times 10^8$ |

These results demonstrate that DFBNP is superior to the commercially available biocide BIT in paper mill liquors, particularly in the rate of kill.

Example 4

Comparison of the activity of DFBNP, the diacetoxy derivative of BNPD (DABNP) and a 3:1 mixture of 5-chloro-2-methyl-3-isothiazolone and 2-methyl-3-isothiazolone (CMI/MI) in coolin water

100 ml volumes of two solutions of each of DFBNP, DABNP and CMI/MI containing 200 and 25 ppm of biocide in a sterile cooling water containing 0.022% $CaCl_2$, 0.14% $MgCl_2$, 0.22% $NaHCO_3$ and 0.1% ethylene glycol and having a pH of 8.5 are prepared and each is inoculated with 1 ml of an 18 hour broth culture of *Pseudomonas aeruginosa.*

A control which is free from biocide is also inoculated in the same fashion. The test solutions and control are held at 30°C and inspected 1, 2 and 4 hours after inoculation. At each inspection a count of surviving bacteria is made. The results are as follows:

| Biocide | Concentration (ppm) | Surviving Cells/ml | | |
|---|---|---|---|---|
| | | 1 hour | 2 hours | 4 hours |
| DFBNP | 200 | $1.5 \times 10^2$ | <10 | <10 |
| | 25 | $6.4 \times 10^4$ | <10 | <10 |
| DABNP | 200 | $2.2 \times 10^7$ | <10 | <10 |
| | 25 | $1.9 \times 10^7$ | $9.0 \times 10^3$ | $1.9 \times 10^2$ |
| CMI/MI | 200 | $1.2 \times 10^7$ | <10 | <10 |
| | 25 | $1.2 \times 10^7$ | $5.5 \times 10^4$ | $3.0 \times 10^2$ |
| Control (no biocide) | | $6.6 \times 10^7$ | $1.3 \times 10^7$ | $5.4 \times 10^7$ |

These results indicate that DFBNP is superior to its closest structural analogue DABNP and also to the commercially available cooling water biocide, CMI/MI particularly in respect of its rate of kill.

Example 6

Comparison of the activity of DFBNP, DABNP and Glutaraldehyde (Nalfloc 4007) against anaerobic bacteria (Secondary Oil Recovery)

An aqueous medium containing the following ingredients is prepared, $KH_2PO_4$: 2.25 g, $NH_4Cl$: 4.5 g, $Na_2SO_4$: 4.5 g, sodium lactate (70%): 22.5 ml, yeast extract: 4.5 g, aged seawater: 3375 ml, and distilled water: 1125 ml and imediately before sterilisation the following reducing agents are added sodium thioglycollate: 0.45 g, sodium ascorbate: 0.45 g and ferrous sulphate: 2.25 g. The pH is adjusted to 7.8 and 9 ml volumes of the solution sterilised at 121°C for 15 minutes. To each volume is added 1 ml of a solution of one of the biocides (DFBNP, DABNP and glutaraldehyde) containing sufficient active ingredient to give a range of final biocide concentrations from 10 ppm to 100 ppm in different samples. Each sample is inoculated with 0.1 ml of a 10-day culture of *Desulphovibrio desulphuricans* (strain T886: Shell), incubated for 5 days at 25°C and assessed for the presence or absence of growth. The minimum inhibitory concentrations (MIC) quoted below are those which prevented growth.

| Biocide | MIC (ppm) |
|---|---|
| DFBNP | 25 |
| DABNP | 25 |
| Glutaraldehyde | 50 |

These results indicate that DFBNP is similar in its activity against anaerobic bacteria to DABNP and superior to glutaraldehyde which is commonly used to combat anaerobic bacteria in secondary oil recovery.

Example 7

Comparison of the activity of DFBNP and DABNP against bacteria in a paper mill liquor

The procedure of Example 3 is repeated except that DABNP is used in place of BIT.

The results are as follows:

| Biocide | Concentration (ppm) | Surviving Cells/ml | | |
|---|---|---|---|---|
| | | 1 hour | 2 hours | 4 hours |
| DFBNP | 200 | <10 | <10 | <10 |
| | 500 | <10 | <10 | <10 |
| DABNP | 200 | $8.2 \times 10^5$ | $2.0 \times 10^4$ | <10 |
| | 500 | $1.4 \times 10^5$ | <10 | <10 |
| Control (no biocide) | | $9.0 \times 10^7$ | $7.2 \times 10^7$ | $8.2 \times 10^7$ |

These results indicate that DFBNP is superior to its closest structural analogue in combating bacteria in a typical paper mill liquor, particularly in respect of the rate of kill.

Example 8

Comparison of the algistatic activity of DFBNP, DABNP, the dipropionyl derivative of BNPD (DPBNP) and Lauryl-benzyl dimethylammonium chloride (LBDAC) against freshwater algae

To 50 ml samples of an algal broth containing 1 ppm of biocide are added 1 ml aliquots of a mixed algal suspension containing *Akystrodesmus augustus, Chlamydomonas reinhardii* and *Stichococcus bacillaris.* The samples are incubated for 1 week at 18°C under artificial illumination and inspected for the presence of algal growth. The results are as follows:

| Biocide | Presence(+)/Absence(—) of algal growth |
|---|---|
| DFBNP | — |
| DABNP | — |
| DPBNP | — |
| LBDAC | — |
| Control (no biocide) | + |

These results indicate that DFBNP is as active as the other compounds in inhibiting the growth of freshwater algae over a period of one week.

**Claims**

1. The compound 1,3-diformyloxy-2-bromo-2-nitro propane (DFBNP).
2. A process for the preparation of DFBNP comprising reacting a mixed anhydride of formic acid and acetic acid, formed *in situ,* with 2-bromo-2-nitro-1,3-propanediol (BDNP) in the presence of a basic catalyst.
3. A process according to Claim 2 wherein the catalyst is pyridine.
4. A method for the protection of a medium against attack by micro-organisms which comprises adding the compound of Claim 1 to the medium.
5. A method according to Claim 4 wherein the quantity of DFBNP is in the range 1 to 5000 parts per million.
6. A method according to Claim 4 or Claim 5 wherein the medium is aqueous.
7. A method according to Claim 6 wherein the medium is selected from the group comprising industrial cooling water, paper mill liquor and swimming bath water.
8. A composition comprising the compound of Claim 1 dissolved in a non-hydroxylic water-miscible organic liquid.
9. An aqueous medium containing dissolved therein from 1 to 5000 ppm of the compound according to Claim 1.

**Revendications**

1. Le 1,3-diformyloxy-2-bromo-2-nitropropane (DFBNP).
2. Procédé de préparation du DFBNP, qui comprend la réaction d'un anhydride mixte d'acide formique et d'acide acétique formé *in situ* avec le 2-bromo-2-nitro-1,3-propanediol (BDNP) en présence d'un catalyseur basique.
3. Procédé suivant la revendication 2, dans lequel le catalyseur est la pyridine.
4. Procédé de protection d'un milieu contre l'attaque par des micro-organismes, qui comprend l'addition du composé suivant la revendication 1 à ce milieu.
5. Procédé suivant la revendication 4, dans lequel la quantité de DFBNP est située dans l'intervalle de 1 à 5000 parties par million.
6. Procédé suivant la revendication 4 ou 5, dans lequel le milieu est aqueux.
7. Procédé suivant la revendication 6, dans lequel le milieu est choisi entre une eau de refroidissement industrielle, de la liqueur de papeterie et de l'eau de bain de natation.
8. Composition qui comprend le composé suivant la revendication 1, en solution dans un liquide organique non hydroxylé miscible à l'eau.
9. Milieu aqueux contenant en solution 1 à 5000 ppm du composé suivant la revendication 1.

**Patentansprüche**

1. Die Verbindung 1,3-Diformyloxy-2-bromo-2-nitro-propan (DFBNP).

2. Verfahren zur Herstellung von DFBNP, bei welchem ein gemischtes Anhydrid von Ameisensäure und Essigsäure, das in situ hergestellt worden ist, mit 2-Bromo-2-nitro-1,3-propandiol (BDNP) in Gegenwart eines basischen Katalysators umgesetzt wird.

3. Verfahren nach Anspruch 2, bei welchem der Katalysator Pyridin ist.

4. Verfahren zum Schützen eines Mediums gegen den Angriff durch Microorganismen, bei welchem dem Medium die Verbindung nach Anspruch 1 zugefügt wird.

5. Verfahren nach Anspruch 4, bei welchem die Menge an DFBNP im Bereich von 1 bis 5000 Teilen je Million liegt.

6. Verfahren nach Anspruch 4 oder 5, bei welchem das Medium ein wäßriges ist.

7. Verfahren nach Anspruch 6, bei welchem das Medium aus der Gruppe ausgewählt ist, die industrielles Kühlwasser, Papiermühlenflüssigkeit und Schwimmbadwasser umfaßt.

8. Zusammensetzung, welche die Verbindung nach Anspruch 1 als Lösung in einer nichthydroxylischen, wassermischbaren organischen Flüssigkeit enthält.

9. Wäßriges Medium, welches 1 bis 5000 ppm der Verbindung nach Anspruch 1 gelöst enthält.